# EUROPÄISCHE PATENTSCHRIFT

(11) **EP 1 963 437 B1**
(45) Veröffentlichungstag und Bekanntmachung des Hinweises auf die Patenterteilung: **10.10.2012**
(21) Anmeldenummer: 06841148.7
(22) Anmeldetag: 22.12.2006
(51) Int. Cl.: A61K 8/25, A61K 8/04, A61Q 19/00, C09D 7/12, C09C 3/00, C09C 1/30, C08K 3/36

(54) **AGGLOMERATPARTIKEL, VERFAHREN ZUR HERSTELLUNG VON NANOKOMPOSITEN SOWIE DEREN VERWENDUNG**
AGGLOMERATE PARTICLES, METHOD FOR PRODUCING NANOCOMPOSITES, AND THE USE THEREOF
PARTICULES D'AGGLOMERAT, PROCEDE DE PRODUCTION DE NANOCOMPOSITES ET LEUR UTILISATION

(30) Priorität: 23.12.2005 DE 102005061965
(43) Veröffentlichungstag der Anmeldung: 03.09.2008
(73) Patentinhaber: Fraunhofer-Gesellschaft zur Förderung der angewandten Forschung e.V., 80686 München (DE)
(72) Erfinder: GELLERMANN, Carsten, 97218 Gerbrunn (DE); WOLTER, Herbert, 97941 Tauberbischofheim (DE); STORCH, Werner, 97204 Höchberg (DE)
(74) Vertreter: Pfenning, Meinig & Partner GbR
(86) Internationale Anmeldenummer: PCT/EP2006/012509
(87) Internationale Veröffentlichungsnummer: WO 2007/076990

(56) Entgegenhaltungen:
- EP-A1- 0 725 037
- EP-A1- 1 433 749
- WO-A-03/004143
- DE-A1- 10 242 039
- DE-A1- 19 601 415

## Beschreibung

Die Erfindung betrifft Agglomeratpartikel, die aus agglomerierten nanoskaligen Primärpartikeln aufgebaut sind sowie ein Verfahren zur Einarbeitung dieser Partikel in eine Polymermatrix und die Verwendung dieser Agglomeratpartikel.

Die Herstellung von Nanopartikeln (< 1 µm Primärpartikelgröße) ist literaturbekannt und gelingt über diverse unterschiedliche Verfahren. So ermöglichen Methoden in der Gasphase (Pyrolyse, PVD, CVD, usw.) die Synthese von mehr oder weniger agglomerierten bzw. aggregierten Partikeln als Pulver. Ungünstig ist dabei, dass für schwer einstellbare, geringe Agglomerations- und Aggregationsgrade die Synthese und Aufarbeitung umso aufwändiger und kostenintensiver ist. Oberflächenmodifizierungen an diesen Partikeln, wie beispielsweise eine Hydrophobierung, Modifizierung mit Wirkstoffen oder Funktionalisierung zur kovalenten Einbindung der Partikel in eine Polymermatrix sind ebenfalls oftmals nur sehr aufwändig und nachträglich möglich und führen in der Regel zu Agglomeraten und Aggregaten.

Um die nanoskaligen Primärpartikel gegenseitig abzuschirmen, werden häufig große Mengen an Additiven, Dispergierhilfsmitteln usw. eingesetzt. Dennoch kann eine Agglomeration/Aggregation der Partikel auch auf diese Weise nicht vollständig vermieden werden. Für viele Anwendungen sind diese Zusätze zudem störend. Weitere Nachteile sind beispielsweise hinsichtlich der Verarbeitung bei den viel zu geringen Dichten (Schütt-, Stampfdichte, usw.) zu sehen (vgl. z.B. Aerosil von Degussa). Die Isolierung von vereinzelten Nanopartikeln ist deshalb schwierig und problematisch.

Um die Probleme der Partikelisolierung unter Vermeidung von Agglomeration/Aggregation zu umgehen, werden oxidische Nanopartikel häufig auf nasschemischem Weg hergestellt, in situ silanisiert und in Dispersion stabilisiert. Die so hergestellten, bis 50 Gew.-% Feststoffgehalt enthaltenen Dispersionen können Hydro- oder Organosole sein. Nachteilig bei den Organosolen ist die große Menge an zu verwendenden (Reaktiv)-Lösungsmitteln, die schwer entfernbar sind und daher in der Regel als Ballast angesehen werden. Diese müssen prozessfreundlich und - je nach Anwendung - für ein bestimmtes Polymer verträglich sein. Außerdem wird von der Anwenderseite ein Partikelprodukt auf Pulverbasis aus verarbeitungstechnischen Gründen in der Regel bevorzugt.

Die EP 1 433 749 offenbart Kieselsäureagglomerate mit einem mittleren Durchmesser 1 bis 500 µm, bestehend aus Aggregaten mit einer Partikelgröße von 100 bis 1000 nm und Primärpartikeln mit einer Partikelgröße von 5 bis 50 nm. Diese Agglomerate sind mit Silanen oberflächenmodifiziert worden siehe D1, Beispiele 1-5).

Die DE 196 01 415 offenbart Granulate auf Basis von pyrogen hergestelltem Siliciumdioxid. Der mittlere Korndurchmesser besagter Granulate beträgt 25 bis 120 µm. Die Granulate werden hergestellt, indem pyrogen hergestelltes Siliciumdioxid (Aerosil 380, 300, Ox50, 130, 200) in Wasser dispergiert, sprühtrocknet und die erhaltenen Granulate bei einer Temperatur von 150 bis 1100 °C tempert. Die nach der Sprühtrocknung erhaltenen Granulate können darüberhinaus oberflächenmodifiziert werden (z.B. mit Silanen). Die erhaltenen Granulate haben einen mittleren Korndurchmesser von 14 bis 53 µm.

Die EP 0 725 037 offenbart Granulate auf der Basis von Siliciumdioxid mit einer mittleren Korngröße von 10 bis 120 µm. Die Granulate werden hergestellt, indem pyrogen hergestelltes Siliciumdioxid (Aerosil 380, 300, Ox50, 130, 200) in Wasser dispergiert, sprühtrocknet und die erhaltenen Granulate bei einer Temperatur von 150 bis 1100 °C tempert. Die nach der Sprühtrocknung erhaltenen Granulate können darüberhinaus oberflächenmodifiziert werden (z.B. mit Silanen). Die erhaltenen Granulate haben einen mittleren Korndurchmesser von 14 bis 53 µm.

Aus der DE 102 42 039 sind Silikonkautschukmassen bekannt, die Granulate auf Basis von pyrogen mittels Flammhydrolyse hergestelltem Siliziumdioxid enthalten; diese Silikonkautschukmassen werden als raumtemperaturvernetzende Einkomponent-Dichtungsmassen sowie selbstnivilierende raumtemperaturvernetztende Silikonkautschuk-Dichtmassen eingesetzt.

Die WO 03/004143 betrifft ein Verfahren zur Granulation in einer zirkulierenden Wirbelschicht, eine Vorrichtung zur Durchführung dieses Verfahrens und nach diesem Verfahren erhaltene Granulate sowie deren Verwendung.

Die EP 0 937 755 offenbart ein Verfahren zur Herstellung von Fällungskieselsäuren, in dem eine Fällungskieselsäuresuspension auf einen pH-Wert von 2 bis 3,9 eingestellt und sprühgetrocknet wird. Die Kieselsäureagglomerate bestehen nicht aus agglomerierten, nanoskaligen Primärpartikeln.

Die EP 0 725 037 offenbart ein Verfahren zur Herstellung von Granulaten auf Basis von pyrogen hergestelltem SiO₂ durch Sprühtrocknung einer wässrigen Dispersion von Aerosil 380 bis 380 °C. Man erhält nach der Sprühtrocknung Sprühgranulate, aber nicht Agglomerate.

Bezüglich der Definitionen der Begriffe "Aggregat" sowie "Agglomerat" wird auf das Römpp Chemie-Lexikon verwiesen. Demnach sind Agglomerate Zusammenballungen aus Primärteilchen, Aggregaten oder einer Mischung aus beiden, deren Zusammenhalt so gering ist, dass sie mit den üblichen Verfahren der Herstellung von Beschichtungsstoffen oder Druckfarben zerteilt werden können. Sie sind über Kanten und Ecken brückenartig miteinander verbunden. Ihre Oberfläche unterscheidet sich nicht wesentlich von der Summe der Oberflächen aller sie aufbauenden Teilchen (DIN 53206-1: 1972-08). Agglomerate von Pigmenten und Füllstoffen entstehen vornehmlich bei der Trocknung im Verlauf der Pigmentherstellung.

Unter Aggregaten werden hingegen ein verwachsener Verband von Pigmentteilchen oder Füllstoffteilchen, der aus flächig aneinandergelagerten Primärteilchen besteht. Die Oberfläche ist kleiner als die Summe der Oberflächen der Primärteilchen (DIN 53206-1: 1972-08). Aggregate lassen sich im Gegensatz zu Agglomeraten durch die üblichen Dispergierverfahren für Pigmente und Füllstoffe nicht zerteilen (DIN EN 13900-1: 2003-06, DIN EN ISO 8780-1: 1995-04).

Ausgehend hiervon ist es deshalb die Aufgabe der vorliegenden Erfindung, Partikel anzugeben, die eine problemlose Weiterverarbeitung zu Nanopartikel enthaltenden Produkten ermöglichen sowie ein Verfahren zur Herstellung von derartigen Kompositen anzugeben.

Die Aufgabe wird in Bezug auf die Partikel durch die Merkmale des Anspruchs 1, in Bezug auf das Verfahren zur Herstellung von Partikeln enthaltenden Polymermatrizes durch die Merkmale des Anspruchs 10 gelöst. Die Unteransprüche zeigen vorteilhafte Weiterbildungen auf.

Gemäß Patentanspruch 1 werden Agglomeratpartikel in fester Form vorgeschlagen, die aus nanoskaligen Primärpartikeln aufgebaut sind. Der Kern der erfindungsgemäßen Lehre ist darin zu sehen, dass für die Verarbeitung von Nanopartikeln nicht die Nanopartikel als solches in Form einer nasschemischen Dispersion zur Verfügung gestellt werden, sondern dass die Nanopartikel in Form von agglomerierten, aber auch gezielt zerkleinerten nanoskaligen Primärpartikeln vorliegen. Diese so agglomerierten nanoskaligen Primärpartikel bilden dann die erfindungsgemäßen Agglomeratpartikel mit einem mittleren Partikeldurchmesser von 0,5 bis 100 µm, bevorzugt mit einem Partikeldurchmesser von 1 bis 20 µm.

Die erfindungsgemäßen Agglomeratpartikel vereinen vorteilhafte Eigenschaften von Mikropartikeln mit denen von Nanopartikeln. Aufgrund der verfahrensgemäßen Agglomeration von Primärnanopartikeln zu mikrometergroßen Agglomeratpartikeln entsprechen deren Eigenschaften denen der klassischen Mikropartikel. Vorteilhaft sind die für die Verarbeitung wichtige geringere Staubneigung, ihre gute Rieselfähigkeit und der im Vergleich zu Nanopartikeln gesundheitliche Aspekt (geringe Lungengängigkeit). Dies ist ein für die Arbeitssicherheit der mit Pulvern umgehenden Arbeiter wesentlicher Vorteil. Hervorzuheben ist in diesem Zusammenhang vor allem auch, dass die neuartigen Agglomeratpartikel im Gegensatz zu anderen Nanopulvern erstmals ohne großen zusätzlichen Aufwand konventionell, d.h. in üblichen Maschinen wie Laborextrudern verarbeitet werden können.

Ein weiterer Vorteil der Erfindung ist in der Minimierung der Oberflächenenergie der Partikel durch die Kombination verschiedener Effekte zu sehen:
- Geometrie
- Chemische Modifizierung
- Anpassung der Partikeloberfläche an Matrix (z.B. gute Benetzbarkeit).

Es ist gelungen, die Berührungsflächen der Primärpartikel zu minimieren, d.h. maximal eine punktuelle Berührung untereinander einzustellen. Dies gelingt dann besonders gut, wenn die Primärpartikel annähernd sphärisch sind.

Bevorzugt sind dabei die Primärpartikel so ausgewählt, dass der gebildete Agglomeratpartikel beim Einarbeiten z.B. unter Einwirkung von Scherkräften wieder in die Nanopartikel zerfällt. Auf diese Weise können somit Nanopartikel in ein Polymer problemlos auch bis zu hohen Füllgraden, nämlich bis zu 40 Gew.-%, eingearbeitet werden.

Die erfindungsgemäßen Agglomeratpartikel zeichnen sich durch ihre einheitliche Form (z.B. kugelförmig oder donutartig, hohl oder kompakt), gutes Fließverhalten, geringe Verdickungswirkung, einfache Verarbeitbarkeit und eine einstellbare Zerkleinerbarkeit, d.h. beispielsweise eine gute Deagglomerierbarkeit mit geringem energetischem Aufwand aus.

Bei den nanoskaligen Primärpartikeln ist es günstig, wenn diese im Wesentlichen die gleiche Korngröße aufweisen, sodass dann auch im Endeffekt nahezu gleichförmige, vorzugsweise sphärische Agglomeratpartikel entstehen. Die Primärpartikel können dabei auch in einer Kern-Schalen-Form oder als Hohlkugeln vorliegen. Ebenso ist es aber auch möglich, dass dreidimensionale, zweidimensionale oder eindimensionale Nanosysteme, z.B. Kugeln, alle Kristallstrukturen, Plättchen, Schichtsilikate und andere Schichtsysteme, Fasern, Tubes, Drähte, usw., eingesetzt werden.

Erfindungsgemäß sind die Primärpartikel ausgewählt aus den folgenden Metallen: Silizium, Zinn, Titan, Zirkon, Calcium, Strontium, Barium, Aluminium, Yttrium, Zink, Tantal, Cer, Gadolinium, Holmium, Erbium, Ytterbium sowie Glas, Glaskeramik und Keramik-bildende Materialien und/oder deren Kombinationen. Das Material kann sowohl kristallin, teilkristallin als auch amorph sein.

Die Agglomeratpartikel können beliebig mit anderen Füllstoffen und/oder Additiven zur Herstellung von Kompositen bzw. Nanokompositen kombiniert werden. Bei den Füllern können dies beliebige Partikel, Fasern im Nano- oder Mikrometerbereich oder Gewebe sein. Beispiele dafür sind klassische Glasfüller, Glasfaser und Gewebe, silikatische Füller, Polykieselsäuren, Metalle und Metalloxide, Polymere (z.B. Beads), Kohlenstoff (Graphit, Ruß, Fullerene, C-Nanotubes, Nano- und Mikrofasern), Bariumsulfat, Siliciumcarbid, Siliciumnitrid, Titanate und Aluminiumhydroxid (ATH). Bei den Additiven können dies verschiedene übliche Stabilisatoren (Inhibitoren, Antioxidantien, Biostabilisatoren, Lichtschutzmittel (UV-Absorber) oder Metalldesaktivatoren), Verarbeitungshilfsmittel (Gleitmittel, Trennmittel, Slip-, Antislip-, Antiblockmittel usw.), mechanische Zusatzstoffe (Weichmacher, Schlagzähmodifizierer, usw.) sowie Produktmodifizierer (Antistatika, Brandhemmstoffe, Keimbildner für teilkristalline Polymere, Treibmittel, optische Aufheller, Pigmente/Farbstoffe usw.).

Bevorzugt ist es bei den erfindungsgemäßen Agglomeratpartikeln, wenn diese aus nanoskaligen Primärpartikeln aufgebaut sind, deren Oberflächen funktionalisiert worden sind. Derartige Agglomeratpartikel, d.h. Agglomeratpartikel, die aus Oberflächen-modifizierten Nanopartikeln gebildet worden sind, zeigen eine ausgezeichnete Desagglomeration, wenn sie z.B. unter Einwirkung von Scherkräften in eine polymere Matrix eingearbeitet werden. Bei der Erfindung ist es deshalb bevorzugt, dass für die Anwendungsfälle, für die eine Desagglomeration der Agglomeratpartikel angestrebt ist, solche verwendet.werden, die aus Oberflächen-modifizierten Primärpartikeln aufgebaut sind.

Weiterhin ist eine chemische Modifizierung zur Einstellung definierter interpartikulärer Wechselwirkungen vorteilhaft. Diese Wechselwirkungen müssen derart ausgeprägt sein, dass die Primärpartikel einerseits punktuell locker zusammengehalten werden und sich andererseits bei Bedarf ohne großen Aufwand (Scherkräfte) zerkleinern lassen. Wesentlich sind dabei die auf der Oberfläche vorhandenen OH-Gruppen - beim SiO₂ sind es beispielsweise Silanolgruppen - zu minimieren, damit eine interpartikuläre irreversible Kondensation und damit eine Aggregatbildung vermieden wird. Als weiterer wesentlicher Aspekt ist die Anpassung der Partikeloberfläche an die Polymermatrix zu nennen.

Erfindungsgemäß können sowohl Nanopartikel-haltige wässrige Hydrosol als auch Organosole als Ausgangsbasis dienen. Diese sind über bekannte Verfahren herstellbar. Nanopartikel aus beispielsweise SiO₂, TiO₂, SnO₂, ZrO₂, usw. haben sich zur Herstellung von zerkleinerbaren Agglomeratpartikeln als erfolgreich erwiesen. Dabei sind die mittleren Partikelgrößen im Bereich von 2 bis 500 nm. Vorzugsweise werden Partikel mit engen Partikelgrößenverteilungen eingesetzt.

Auch kommerzielle Sole wie beispielsweise ein Kieselsol oder ein wässriges ZrO₂-Sol können eingesetzt werden. Als Lösungsmittel dienen dabei reines Wasser oder auch hydrophile, hydrophobe, protonische oder aprotonische organische Lösungsmittel. Unter den organischen Lösungsmitteln sind Alkohole bzw. Ketone bevorzugt. Ein-, zwei- und dreiwertige Alkohole sind geeignet. Als dreiwertiger Alkohol geeignet ist z.B. Glycerin und als zweiwertige Alkohole eignen sich z.B. Ethylenglycol oder die Propandiole. Besonders geeignet sind die einwertigen Alkohole, insbesondere solche mit bis zu fünf C-Atomen im Molekül. Mischungen von Wasser mit organischen Verbindungen werden bevorzugt. Insbesondere bevorzugt werden Aceton, tert.-Butylalkohol, Methanol, Ethanol sowie n- und i-Propanol.

Eine im Hinblick auf die Zerkleinerbarkeit der herzustellenden Agglomeratpartikel notwendige Oberflächenmodifizierung der Primärpartikel gelingt durch eine Kombination der beschriebenen Sole mit speziellen chemischen Substanzen wie Silanen, Silanzane, Siloxanen, Carbonsäuren, Alkoholen, Aminen, Aminosäuren, Sulfonaten, (Poly)phosphaten, Ampholyten, thermoplastischen Homopolymeren, (Block)copolymeren und/oder deren Kombinationen. Besonders vorteilhaft für oxidische Primärpartikel haben sich Silane erwiesen. Diese unterscheiden sich in der Art und Anzahl ihrer hydrolysierbaren Gruppen. Bevorzugte Silane sind Alkoxysilane, insbesondere Moni-, Di-, Trialkoxy- oder - chlorsilane, wie Trimethylchlorsilan, Trimethylmethoxysilan, Dimethyldichlorsilan, Dimethyldimethoxysilan, 3-Aminopropyltrimethoxysilan, 3-Aminopropyltriethoxysilan, 3-Chlorpropyltrimethoxysilan, 3-Chlorpropyltriethoxysilan, 2-Aminoethyl-3-aminopropyl-trimethoxysilan, 3-Glycidyloxypropyltrimethoxysilan, 3-Glycidyloxypropyltriethoxysilan, Hexadecyltrimethoxysilan, iso-Butyltrimethoxysilan, isoButyltriethoxysilan, 3-Mercaptopropyltrimethoxysilan, 3-Methacryloxypropyltrimethoxysilan, 3-Methacryloxypropyltriethoxysilan, Methyltrimethoxysilan, Methyltriethoxysilan, Octyltrimethoxysilan, Octyltriethoxysilan, Pentyltrimethoxysilan, Propyltrimethoxysilan, Propyltriethoxysilan, (2-Aminoethyl)-2-aminoethyl-3-amino-propyltrimethoxysilan, Vinyltrimethoxysilan, Vinyltriethoxysilan, Tetramethoxysilan, Tetraethoxysilan.

Häufig werden dabei besonders gute Effekte bezüglich der Zerkleinerbarkeit der Agglomeratpartikel durch gezielte Kombinationen einzelner chemischer Substanzen ermöglicht. Günstig herausgestellt hat sich beispielsweise die Kombination eines Silans mit einer anderen chemischen Substanz.

Vorzugsweise weisen die Silane eine organofunktionelle Gruppe auf, welche nach der Oberflächenmodifizierung zur Umsetzung bzw. Reaktion mit entsprechenden Partnern wie der Polymermatrix zur Verfügung stehen. Derartige funktionelle Gruppen sind beispielsweise Acryl bzw. Methacryl, Vinyl, Amino, Mercapto, substituierte Carboxygruppen, Alkylgruppen, insbesondere Cl- bis C20-Alkylgruppen, Alkoxygruppen, Cyanogruppen, Isocyanogruppen, Cyanatogruppen, Isocyanatogruppen, Säureanhydride, insbesondere cyclische Anhydride wie Bernsteinsäureanhydrid, Epoxygruppe wie insbesondere Etoxygruppen wie Glycidoxygruppen, Carbamate, etc. Derartige Reaktionspartner ermöglichen erst eine optimale Anpassung der Partikeloberflächen an die Polymermatrix sowie eine im Hinblick auf die mechanischen Eigenschaften der resultierenden Nanokomposite sinnvolle kovalente Anbindung.

Die für die Oberflächenmodifikation verwendbaren chemischen Substanzen werden unter speziellen Synthesebedingungen auf die Partikeloberflächen der Primärpartikel unter Kondensation aufgebracht. Eine Nachkondensation findet dabei während des Trocknungsprozesses statt. Wesentliche Parameter bei der Oberflächenmodifizierung sind vor allem Art und Typ der Reaktanden, Reaktionsmedium, der pH-Wert und die Temperatur sowie die bei der Pulverisolierung verwendeten Trocknungsbedingungen.

In der Regel sind die resultierenden Agglomeratpartikel oxidischer Natur und enthalten im Wesentlichen Sauerstoff. Statt oder neben dem Sauerstoff können auch andere Elemente in den Anionen enthalten sein wie beispielsweise Wasserstoff (z. B. als Hydroxid), Schwefel (z.B. Sulfid, Sulfit, Sulfat), Phosphor (z.B. Phosphat), Halogene (z.B. Perchlorat, Chlorat). Sie können auch Glas, Glaskeramik und Keramik-bildende Materialien enthalten. Das Material kann sowohl kristallin, teilkristallin als auch amorph sein. Die Morphologie der Agglomeratpartikel ist im Hinblick auf die Zerkleinerbarkeit nicht relevant und daher unkritisch. Da diese Partikel aus Primärpartikeln aufgebaut sind, enthalten sie dazwischen Hohlräume (Zwickel), die für eine definierte Porosität verantwortlich sind. Diese ist unabhängig von der Primärpartikelgröße, sondern hängt viel mehr von der Oberflächenenergie und damit auch von den Wechselwirkungen zwischen den Partikeln ab.

Nach der Zerkleinerung in den aus der Kunststoffindustrie bekannten Maschinen (z.B. Extrudern) werden unter Scherkrafteinwirkung Nanokomposite erhalten, die letztendlich die vorteilhaften Eigenschaften der über andere Verfahren aufwendig hergestellten Nanokomposite realisiert werden.

Untersuchungen zur Zerkleinerbarkeit wurden in wässrigen und organischen Lösungsmitteln durchgeführt. Dazu wurde eine definierte Menge an Agglomeratpartikeln, in z.B. Ethanol gegeben und über einen Zeitraum von bis zu 30 min. einer Ultraschallbehandlung ausgesetzt. Die durch diese Behandlung eingetragene Energie ist relativ gering im Vergleich zu den im Laborextruder mit Doppelschnecken erzeugten starken Scherkräften und dem damit verbundenen hohen Gesamtenergieeintrag. Daher sollten die im Labor mittels Ultraschall bereits schnell zerkleinerbaren Agglomeratpartikel im Extruder ebenfalls gut zerkleinerbar sein.

Die Erfindung betrifft weiterhin ein Verfahren zur Einarbeitung von Agglomeratpartikeln, wie vorstehend beschrieben, in eine Polymermatrix. Es ist hierbei besonders hervorzuheben, dass es mit den erfindungsgemäßen Agglomeratpartikeln möglich ist, bis zu 40 Gew.-%, bezogen auf die Gesamtmasse der Polymermatrix, einzuarbeiten. Die Einarbeitung der Agglomeratpartikel in die Polymermatrix kann dabei auf Agglomeratpartikeln, die in fester Form vorliegen, in der Weise erfolgen, dass diese direkt in eine entsprechende Vorrichtung eingegeben werden, z.B. in einen Extruder, oder aber dass die Agglomeratpartikel in einem Masterbatch vorliegen und dieser Masterbatch dann nach bekannten Verfahren mit der Polymermatrix kombiniert wird.

Die gezielte/kontrollierte Agglomeration der Primärnanopartikel zu den Agglomeratpartikeln kann gemäß der vorliegenden Erfindung nach an und für sich bekannten Verfahren, wie Sprühtrocknung, Sprühgefriertrocknung, Gefriertrocknung und/oder Aerosolverfahren erfolgen.

Wenn die Agglomeratpartikel für eine Verarbeitung vorgesehen sind, bei der sie in Nanopartikel zerfallen sollen, hat es sich als günstig erwiesen, wenn dann die Agglomeratpartikel aus Oberflächen-funktionalisierten Primärpartikeln aufgebaut sind, wobei hier besonders bevorzugt Silan-modifizierte Primärpartikel eingesetzt werden. Als geeignete Verfahren zur Einarbeitung haben sich insbesondere alle Verfahren erwiesen, bei denen die Nanopartikel durch Scherkräfte in die Polymermatrix eingearbeitet werden. In diesem Falle kommt es dann zu einem Zerfall der Agglomeratpartikel zu Nanopartikel. Selbstverständlich ist es hierbei möglich, dass zusätzlich zur Einwirkung von Scherkräften auch noch eine separate Ultraschallbehandlung durchgeführt wird.

Die Agglomeratpartikel bewirken nach ihrer Einarbeitung im Polymer während der Zerkleinerung zu kleineren Agglomeratpartikeln unter Scherung beispielsweise im Laborextruder (Untereinheiten) je nach Partikelgehalt und Oberflächen-Matrix-Adaption eine geringe bis hohe Viskositätssteigerung im Polymer. Bei einer Matrix-angepassten Partikeloberfläche ist auch bei hohen Gehalten bis ca. 40 Gew.-% nur eine geringe Viskositätssteigerung beobachtet worden. Intermediär kann es allerdings zu einem Viskositätsanstieg kommen, der sich hinsichtlich der Zerkleinerung der Agglomerate als vorteilhaft erwiesen hat.

Die resultierenden Nanokomposite (z.B. aus PMMA) zeichnen sich durch Nanopartikel-bedingte Steigerungen beispielsweise des E-Moduls und der Kratzfestigkeit bei im Wesentlichen unveränderter Transparenz und Schlagzähigkeit aus. Weitere in der Regel durch die Partikel vorteilhaft veränderten Eigenschaften betreffen beispielsweise die Härte, Reibungsverhalten, Spannungsrissbeständigkeit, Steifheit, Festigkeiten, thermische Eigenschaften wie Längenausdehnung, Brandverhalten, Wärmeleitfähigkeit; Beständigkeit gegen Umwelteinflüsse wie beispielsweise gegen Licht und Temperaturwechsel; Gas- und Wasserdampfdurchlässigkeit sowie optische Eigenschaften wie Glanz.

Neben der vorstehend beschriebenen Einarbeitung der Agglomeratpartikel in eine Polymermatrix hat es sich gezeigt, dass die erfindungsgemäßen Agglomeratpartikel auch für viele weitere Anwendungen geeignet sind. Allgemein sind die erfindungsgemäßen Agglomeratpartikel geeignet zur Herstellung von Nanodispersionen, Nanokompositen und/oder Nanobeschichtungen. Bevorzugte Anwendung ist die Herstellung von Lacken, Farben, Tinten, Beschichtungssystemen, Flammschutzsystemen und/oder elektrorheologischen Flüssigkeiten. Die Agglomeratpartikel sind auch geeignet zur Herstellung von Kompositen für dielektrische, elektrooptische, und elektrische Applikationen sowie medizinische Anwendungen und als kosmetische, pharmazeutische oder medizinische Hilfsstoffe.

Die Erfindung wird nachfolgend anhand von Ausführungsbeispielen und den Figuren 1 bis 10 näher beschrieben.
Figur 1 zeigt den Aufbau eines erfindungsgemäßen Agglomeratpartikels.
Figur 2 zeigt ein Prinzipschema der Agglomeratbildung und der Desagglomeration.
Die Figuren 3 bis 5 zeigen REM-Aufnahmen von erfindungsgemäßen Agglomeratpartikeln.
Figuren 6a und 6b zeigen dabei TEM-Aufnahmen eines 20 Gew.-% Partikel-haltigen PMMA-Prüfstabes.
Figur 7 zeigt das Verhalten des E-Moduls eines PMMA-Prüfstabes in Abhängigkeit vom Füllgrad mit Agglomeratpartikel.
Figur 8 zeigt die entsprechende Änderung der Vickers-Härte.
Figur 9 zeigt die Untersuchungsergebnisse zur Abrassion mittels Taber-Abraser-Methode.
Figur 10 zeigt UV/VIS-Messungen im Wellenlängenbereich von 400 bis 100 nm einer mit bis zu 20 Gew.-% gefüllten PMMA-Scheibe.

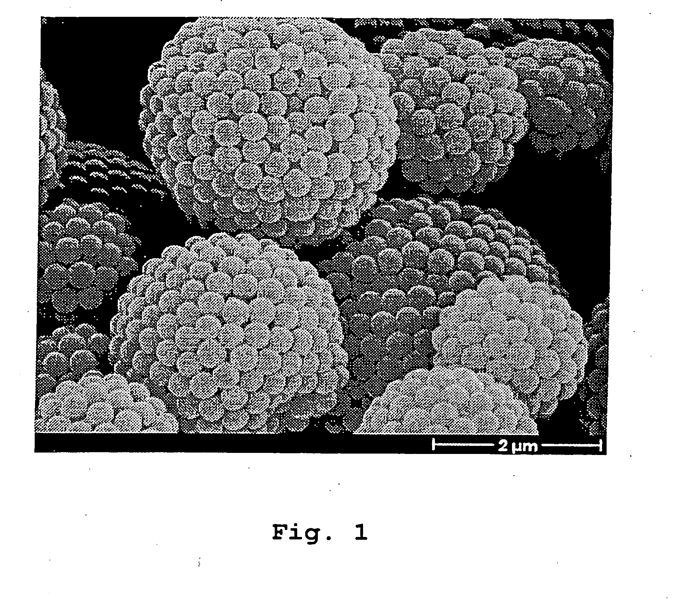

Figur 1 zeigt nun den erfindungsgemäßen Aufbau eines Agglomeratpartikels. Der Agglomeratpartikel nach Figur 1 ist aus einer Vielzahl von einzelnen Primärpartikeln aufgebaut. Als Primärpartikel sind SiO₂-Partikel verwendet worden. Der mittlere Partikeldurchmesser beträgt 3 µm.

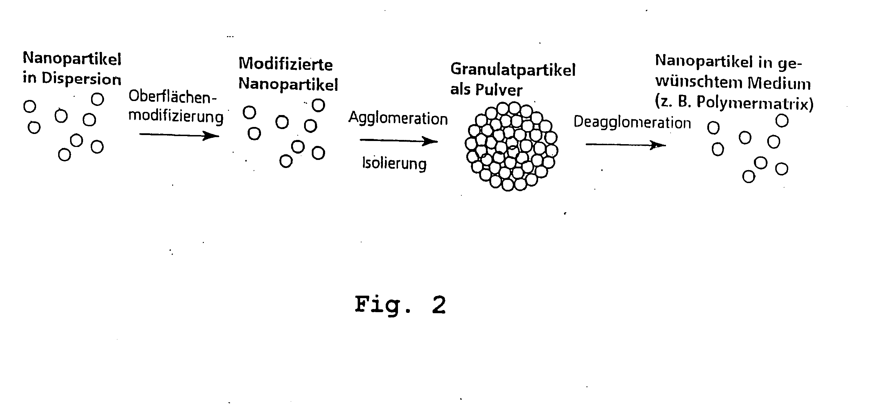

Figur 2 zeigt nun das Prinzipschema zur Modifizierung, Agglomeration und Isolierung von dispergierten Nanopartikeln in Form von Agglomeratpartikeln und den Desagglomerationen in einem Dispergiermedium.

Nachfolgend wird die Erfindung durch Beispiele näher erläutert.

### 1. Kugelförmiges Agglomerat basierend auf SiO₂

Zu 100 ml eines kommerziellen 40 Gew.-%igen Kieselsols (Ludox, Fa. DuPont) wurden langsam und unter starkem Rühren 5 g Aerosil 380 und gegebenenfalls Alkohol hinzugegeben. Gegebenenfalls wird mit 50 ml Wasser verdünnt, um ein sprühfähiges Sol einzustellen. Anschließend wird eine Sprühtrocknung mit einem Mini Spry Dryer Modell 190 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 180 °C
- Ausgangstemperatur: 76 °C
- Pumpenleistung: 70 %
- Aspiratoreinstellung: 100 %

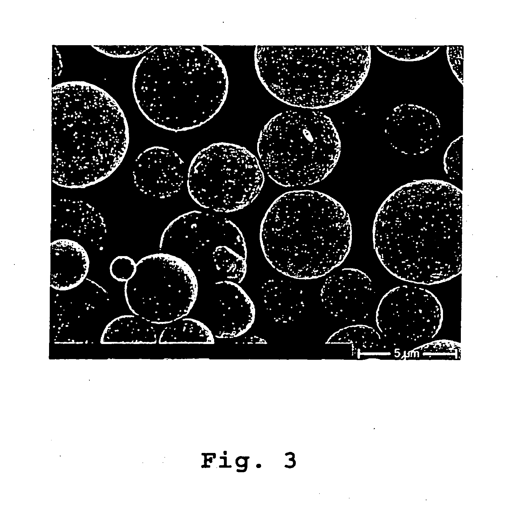

Es resultierten 46 g eines weißen Pulvers mit einer mittels Fraunhofer-Beugung gemessenen, mittleren Partikelgröße von 3 µm. Die Partikel sind relativ kompakt, weisen aber aufgrund der Partikel-basierenden Synthese eine gewisse Porosität auf (keine Hohlpartikel). Der Test zur Desagglomeration (Dispergierung in Ethanol mittels Ultraschall und anschließender Messung mittels dynamischer Lichtstreuung) ergab eine mittlere Partikelgröße von 2,4 µm (Figur 3).

### 2. Kugelförmiges Agglomerat basierend auf SiO₂ und SnO₂

50 g eines kommerziellen 40 Gew.-%igen Kieselsols (Ludox, Fa. DuPont) und 133 Gew.-% einer SnO₂-Dispersion (15 Gew.-% SnO₂, Fa. Alfa Aesar, enthaltend SnO₂-Partikel der Größe 15 nm) wurden unter starkem Rühren zusammengegeben. Gegebenenfalls wird mit 50 ml Wasser verdünnt, um ein sprühfähiges Sol einzustellen. Anschließend wird eine Sprühtrocknung mit einem Mini Spry Dryer Modell 190 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 180 °C
- Ausgangstemperatur: 88 °C
- Pumpenleistung: 30 %
- Aspiratoreinstellung: 100 %

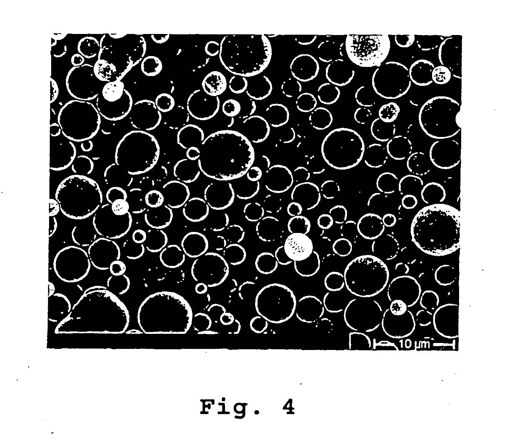

Es resultierten 31 g eines weißen Pulvers mit einer mittels Fraunhofer-Beugung gemessenen, mittleren Partikelgröße von 4 µm. Die Röntgenfluoreszenzanalyse ergab einen SnO₂-Gehalt von 47 Gew.-%. Der Test zur Desagglomeration (Dispergierung in Ethanol mittels Ultraschall und anschließender Messung mittels dynamischer Lichtstreuung) verlief negativ, ergab eine mittlere Partikelgröße von 2,6 µm (Figur 4).

### 3. Ringförmiges Agglomerat basierend auf SiO₂

10 ml eines kommerziellen 40 Gew.-%igen Kieselsols (Ludox, Fa. DuPont) wurden mit 90 ml Wasser versetzt. Anschließend wird eine Sprühtrocknung mit einem Mini Spry Dryer Modell 190 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 220 °C
- Ausgangstemperatur: 136 °C
- Pumpenleistung: 10 %
- Aspiratoreinstellung: 100 %

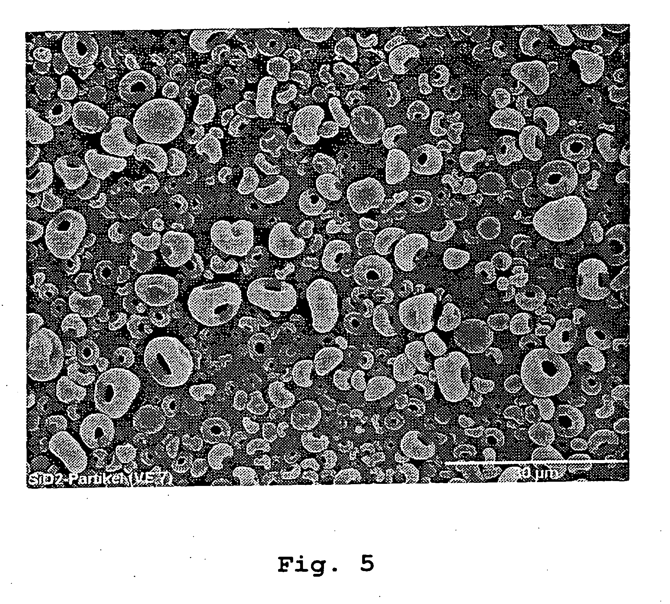

Es resultierten 2 g eines weißen Pulvers mit einer mittels Fraunhofer-Beugung gemessenen, mittleren Partikelgröße von 7 µm. Der Test zur Desagglomeration (Dispergierung in Ethanol mittels Ultraschall und anschließender Messung mittels dynamischer Lichtstreuung) verlief negativ, ergab eine mittlere Partikelgröße von 5,8 µm (Figur 5).

### 4. Agglomerat bestehend aus Methacryl-modifiziertem SiO₂

125 g eines kommerziellen Kieselsols (Köstrosol 2040, Fa. Chemiewerk Bad Köstritz) enthaltend SiO₂-Partikel der Größe 25 nm) wurden mit 45 g Wasser versetzt. Weiterhin wurden langsam 5,8 g (3-Methacryloxy)propyltrimethoxysilan und gegebenenfalls Alkohol hinzugegeben und unter ständigem Rühren über einen Zeitraum von 6 h refluxiert (Ölbadtemperatur: 120 °C). Da der resultierende Ansatz sehr viskos ist, wird mit ca. 100 ml Wasser verdünnt, um ein sprühfähiges Sol einzustellen. Anschließend wird eine Sprühtrocknung mit einem Mini Spry Dryer Modell B-191 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 130 °C
- Ausgangstemperatur: 80 °C
- Pumpenleistung: 2
- Aspiratoreinstellung: 2

Es resultierten 37 g eines weißen Pulvers mit einer mittels Fraunhofer-Beugung gemessenen, mittleren Partikelgröße von 6 µm. Der Test zur Desagglomeration (Dispergierung in Ethanol mittels Ultraschall und anschließender Messung mittels dynamischer Lichtstreuung) verlief positiv und ergab eine mittlere Partikelgröße von 40 nm. Der Nachweis vorhandener Methacrylgruppen-erfolgte mit Hilfe der diffusen Reflexion FTIR-Spektroskopie (DRIFT) anhand von Signalen der C=O-Streckschwingungen zwischen 1700 und 1750 cm⁻¹.

### 5. Agglomerat bestehend aus Methyacryl-modifiziertem SnO₂

70 g einer kommerziellen SnO₂-Dispersion (15 Gew.-% SnO₂ in H₂O, Fa. Alfa Aesar, enthaltend SnO₂-Partikel der Größe 15 nm) wurden mit 30 g Wasser versetzt. Weiterhin wurden langsam 3,2 g (3-Methyacryloxy)propyltrimethoxysilan und 200 ml Methanol hinzugegeben und unter ständigem Rühren über einen Zeitraum on 6 h refluxiert (Ölbadtemperatur: 120 °C). Da der resultierende Ansatz sehr viskos ist, wird gegebenenfalls mit ca. 50 ml Wasser verdünnt, um ein sprühfähiges Sol einzustellen. Anschließend wird eine Sprühtrocknung mit einem Mini Spry Dryer Modell B-191 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 130 °C
- Ausgangstemperatur: 80 °C
- Pumpenleistung: 2
- Aspiratoreinstellung: 2

Es resultierten ca. 8 g eines weißen Pulvers mit einer mittels Fraunhofer-Beugung gemessenen, mittleren Partikelgröße von 5 µm. Der Test zur Desagglomeration (Dispergierung in Lösungsmittel mittels Ultraschall und anschließender Messung mittels dynamischer Lichtstreuung) verlief positiv und ergab eine mittlere Partikelgröße von 30 nm. Der Nachweis vorhandener Methacrylgruppen erfolgte mit Hilfe der diffusen Reflexion FTIR-Spektroskopie (DRIFT) anhand von Signalen der C=O-Streckschwingungen zwischen 1700 und 1750 cm⁻¹.

### 6. Komposit-Agglomerat bestehend aus SiO₂ und Polyacrylsäure

125 g eines kommerziellen Kieselsols (Köstrosol 2040, Fa. Chemiewerk Bad Köstritz, enthaltend SiO₂-Partikel der Größe 25 nm) wurden mit 45 g Wasser versetzt. Weiterhin wurden langsam 20 g Polyacrylsäure hinzugegeben und unter ständigem Rühren über einen Zeitraum von 6 h refluxiert (Ölbadtemperatur: 120 °C). Gegebenenfalls wurde mit ca. 100 ml Wasser verdünnt, um ein sprühfähiges Sol einzustellen. Anschließend wird eine Sprühtrocknung mit einem Mini Spry Dryer Modell B-191 der Fa. Büchi durchgeführt. Folgende Einstellungen wurden dabei gewählt:
- Zweistoffdüsendurchmesser: 0,5 µm
- Zerstäubermedium: Luft
- Eingangstemperatur: 130 °C
- Ausgangstemperatur: 80 °C
- Pumpenleistung: 2
- Aspiratoreinstellung: 2

Es resultierten 25 g eines weißen Pulvers mit einer mittels Fraunhofer-Beugung gemessenen, mittleren Partikelgröße von 8 µm. Der Test zur Desagglomeration (Dispergierung in Ethanol mittels Ultraschall und anschließender Messung mittels dynamischer Lichtstreuung) ergab eine mittlere Partikelgröße von 640 nm. Der Nachweis vorhandener Methacrylgruppen erfolgte mit Hilfe der diffusen Reflexion FTIR-Spektroskopie (DRIFT) anhand von intensiven Signalen der C=O-Streckschwingungen zwischen 1700 und 1750 cm⁻¹.

### Beispiele zur Einarbeitung von Agglomeratpartikeln in Polymethylmethacrylat (PMMA)

### Kompositbeispiel:

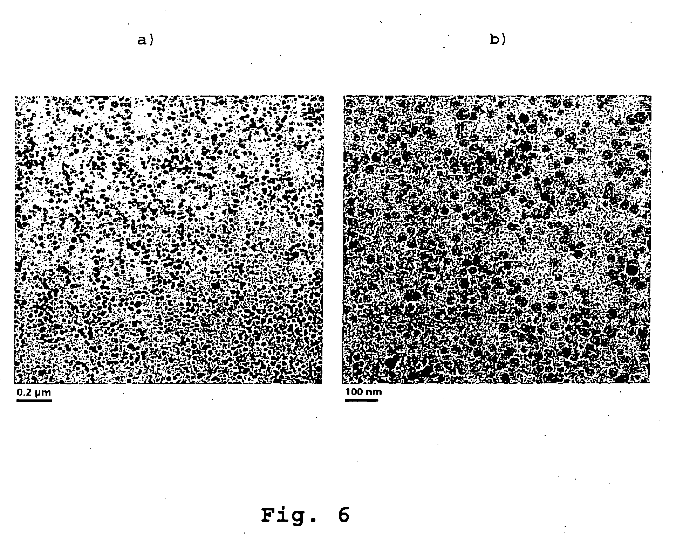

Agglomeratpartikel aus Methacryl-modifiziertem SiO₂ (s. Beispiel 4) wurden mit einem Doppelschneckenextruder (Modell ZK 25T, Fa. Collin) in verschiedenen Verhältnissen bis zu 26 Gew.-% in thermoplastisches Polymethylmethacrylat (PMMA 8N, Fa Röhm) eingearbeitet. Zunächst wurde ein Masterbatch hergestellt, das später zur Verdünnung mit PMMA verwendet wurde. Zur Masterbatch-Herstellung wurde das Material dreimal, d.h. je einmal zur Einarbeitung, Homogenisierung und Entgasung, durch die kleine Verarbeitungsmaschine extrudiert. Eine vierte Extrusion erfolgte zur Einstellung der unterschiedlichen Gehalte durch Verdünnung mit reinem PMMA. Für die Charakterisierung wurden sowohl Prüfstäbe (Mechanik), Folien (Abrasion) und Scheibchen (Transparenz) hergestellt. Die in Figur 6a und b gezeigten TEM-Aufnahmen der resultierenden, 20 Gew.-% SiO₂ enthaltenen PMMA-basierenden Nanokomposite zeigen eindrucksvoll, dass die Scherkräfte des Doppelschneckenextruders offensichtlich ausreichen, die mittels Sprühtrocknung hergestellten Agglomeratpartikel in die Einzelbestandteile (Primärpartikel) zu zerlegen.

Die Eigenschaften des PMMA ließen sich durch den Einbau der Methacryl-modifizierten SiO₂-Partikel in Form der Agglomeratpartikel steigern, wie folgende Beispiele zeigen.

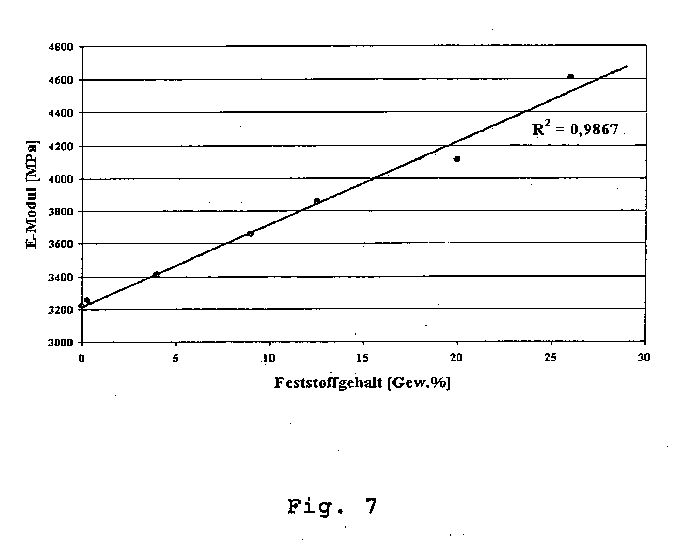

Die Füllung von PMMA mit bis zu 26 Gew.-% Agglomeratpartikeln bewirkt einen Anstieg des E-Moduls um bis zu 43 % (s. Figur 7).

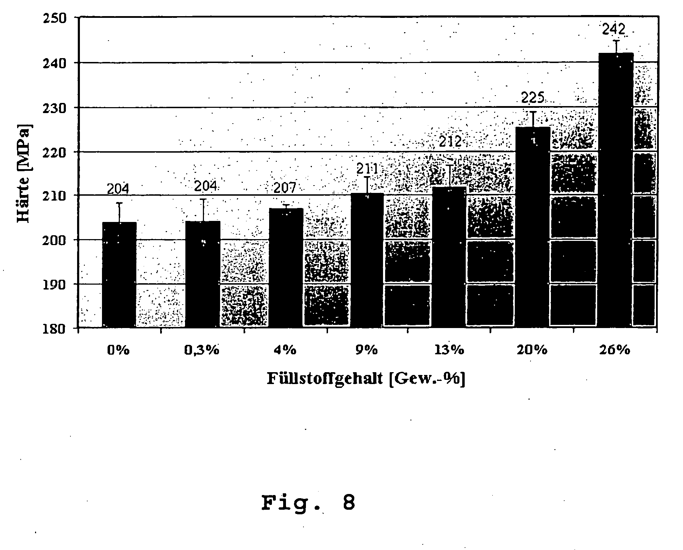

Gleichzeitig zeigt sich bei der Vickers-Härte eine Steigerung um 19 % (s. Figur 8).

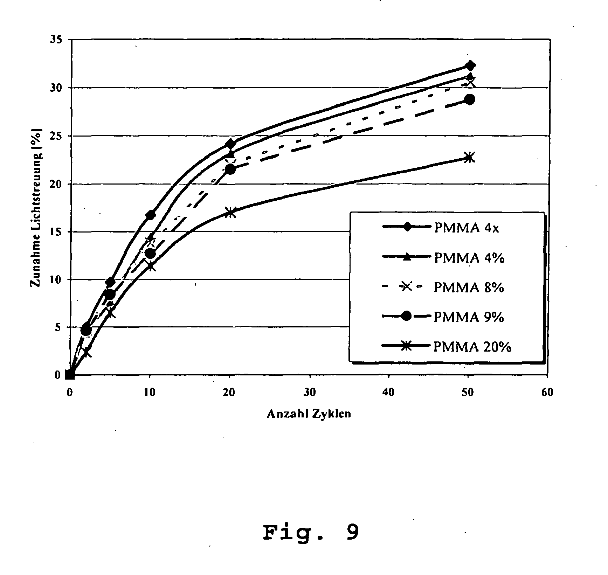

Untersuchungen zur Abrasion mittels Taber-Abraser-Methode (50 Zyklen) bestätigen, dass die Füllung von PMMA mit 20 Gew.-% Partikeln eine Abnahme der Lichtstreuung um 30 % bewirkt (s. Figur 9).

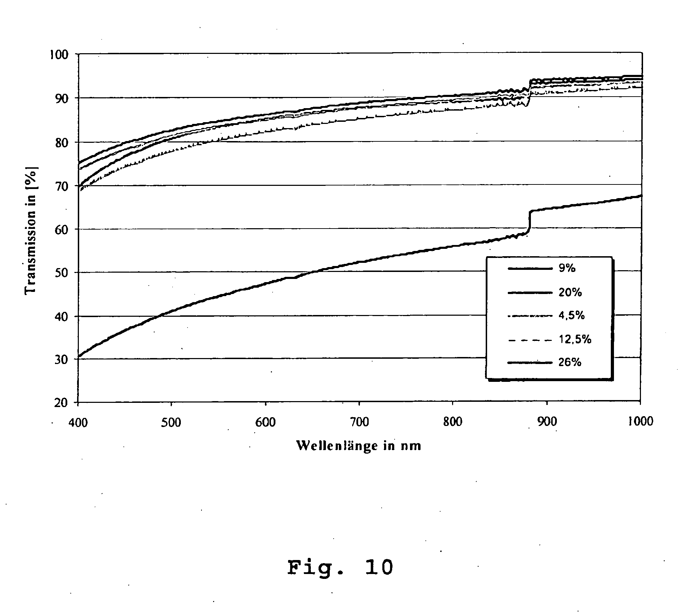

UV/VIS-Messungen im Wellenlängenbereich von 400 bis 1000 nm zeigen die vergleichsweise hohe Transmission der bis zu 20 Gew.-% gefüllten PMMA-Scheibchen und bestätigen damit den hohen Desagglomerationsgrad des Granulatpulvers (s. Figur 10).

### Charakterisierung hinsichtlich Desagglomeration

Zur Ermittlung des Desagglomerationsgrades wurden je 0,5 g des resultierenden Pulvers (Agglomeratpartikel) in 10 g eines Lösungsmittels (Wasser bzw. Ethanol) gegeben und mittels Ultraschall behandelt. Dazu wurde ein Ultraschallstab verwendet, der direkt in die Dispersion getaucht wurde. Es wurde eine zeitabhängige Größenbestimmung der Granulate mittels Fraunhofer-Beugung und dynamischer Lichtstreuung (Photonenkorrelationsspektroskopie) durchgeführt. Dazu wurde nach definierten Zeitabständen (10, 20 und 30 min) ein geringer Anteil der Dispersion entnommen. Es stellte sich heraus, dass je nach Oberflächenmodifizierung der eingesetzten Nanopartikel und in Abhängigkeit vom Herstellungsprozess eine Desagglomeration einstellbar ist. D.h. bei einzelnen Proben wurden mittlere Durchmesser von unter 100 nm gemessen.

## Patentansprüche

1. Agglomeratpartikel mit einem mittleren Partikeldurchmesser von 0,5 bis 100 µm bestehend aus agglomerierten nanoskaligen Primärpartikeln ausgewählt aus Oxiden der folgenden Elemente: Silizium, Zinn, Titan, Zirkon, Calcium, Strontium, Barium, Aluminium, Yttrium, Zink, Tantal, Cer, Gadolinium, Holmium, Erbium, Ytterbium sowie Glas, Glaskeramik und Keramik-bildende Materialien und/oder deren Kombinationen, mit einer mittleren Korngröße im Bereich von 2 bis 500 nm.

2. Agglomeratpartikel nach Anspruch 1, **dadurch gekennzeichnet, dass** sie sphärisch, bevorzugt kugelförmig sind.

3. Agglomeratpartikel nach Anspruch 1 oder 2,
**dadurch gekennzeichnet, dass** die nanoskaligen Primärpartikel sphärisch, bevorzugt kugelförmig sind.

4. Agglomeratpartikel nach mindestens einem der Ansprüche 1 bis 3,
**dadurch gekennzeichnet, dass** die nanoskaligen Primärpartikel im Wesentlichen die gleiche Korngröße aufweisen.

5. Agglomeratpartikel nach mindestens einem der Ansprüche 1 bis 4,
**dadurch gekennzeichnet, dass** der mittlere Partikeldurchmesser der Agglomeratpartikel im Bereich von 1 bis 20 µm liegt.

6. Agglomeratpartikel nach mindestens einem der Ansprüche 1 bis 5,
**dadurch gekennzeichnet, dass** die nanoskaligen Primärpartikel in ihrer Oberfläche funktionalisiert sind.

7. Agglomeratpartikel nach Anspruch 6,
**dadurch gekennzeichnet, dass** die Oberfläche mit Silanen, Silazanen, Siloxanen, Carbonsäuren, Alkoholen, Aminen, Aminosäuren, Sulfonaten, (Poly)phosphaten, Ampholyten, thermoplastischen Homopolymeren, (Block)copolymeren und/oder deren Kombinationen modifiziert ist.

8. Agglomeratpartikel nach mindestens einem der Ansprüche 1 bis 7,
**dadurch gekennzeichnet, dass** die Agglomeratpartikel hohl oder kompakt sind.

9. Verfahren zur Einarbeitung von Agglomeratpartikeln nach einem der vorhergehenden Ansprüche in eine Polymermatrix,
**dadurch gekennzeichnet, dass** durch Agglomeration von nanoskaligen Primärpartikeln mit einem Partikeldurchmesser von 0,5 bis 100 nm gebildete Agglomeratpartikel mit einem Partikeldurchmesser von 0,5 bis 50 µm, in fester Form, in die Polymermatrix eingearbeitet werden.

10. Verfahren nach Anspruch 9,
**dadurch gekennzeichnet, dass** die Agglomeratpartikel einen Durchmesser von 1 bis 20 µm aufweisen.

11. Verfahren nach Anspruch 9 oder 10,
**dadurch gekennzeichnet, dass** bis zu 40 Gew.-%, bevorzugt bis zu 25 Gew.-%, Agglomeratpartikel,
bezogen auf die Gesamtmasse der Polymermatrix, eingearbeitet werden.

12. Verfahren nach mindestens einem der Ansprüche 9 bis 11,
**dadurch gekennzeichnet, dass** die Agglomeratpartikel oder deren Agglomerate in einem Masterbatch vorliegen.

13. Verfahren nach mindestens einem der Ansprüche 9 bis 12,
**dadurch gekennzeichnet, dass** die Agglomeratpartikel so gewählt sind, dass sie bei der Einarbeitung in die Polymermatrix oder einer Polymervorstufe durch Scherkräfte in die nanoskaligen Primärpartikel zerfallen.

14. Verfahren nach Anspruch 13,
**dadurch gekennzeichnet, dass** zusätzlich ein Energieeintrag z.B. durch Ultraschall erfolgt.

15. Verfahren nach mindestens einem der Ansprüche 9 oder 14,
**dadurch gekennzeichnet, dass** die Einarbeitung der Agglomeratpartikel mittels Mischer, Kneter, Dissolver, oder mittels Extrusion in einem Extruder erfolgt.

16. Verfahren nach einem der Ansprüche 13 bis 15,
**dadurch gekennzeichnet, dass** die Agglomeratpartikel durch Agglomeration von oberflächenfunktionalisierten, nanoskaligen Primärpartikeln gebildet werden.

17. Verfahren nach mindestens einem der Ansprüche 9 bis 16,
**dadurch gekennzeichnet, dass** die Agglomeration der Primärpartikel zu den Agglomeratpartikeln durch Sprühtrocknung, Sprühgefriertrocknung, Gefriertrocknung und/oder Aerosolverfahren erfolgt.

18. Verfahren nach mindestens einem der Ansprüche 9 bis 17,
**dadurch gekennzeichnet, dass** die Polymermatrix ausgewählt ist aus Polyamiden, Polyurethanen, Polyestern (gesättigte, ungesättigte und Polycarbonate), Polyacrylnitrilen, Butadien- oder Isoprenpolymerisaten, Polystyrolen, Polypropylen, Polyethylen, Polyvinylchlorid, Poly(meth)-acrylaten, Polyethern, Siliconen, anorganischorganischen Hybridpolymeren (ORMOCERen), nichtschrumpfenden bzw. expandierenden Polymeren wie Spiroorthoestern, elektrisch leitfähigen Polymeren, Aminoplasten, Melaminharzen, Polysulfonen, Polyimiden, deren Kombinationen zu Copolymeren, Blends oder daraus abgeleiteten Kompositen und Lösungsmittel- oder Reaktivmonomer-haltigen (Lack)-Systemen, die gegebenenfalls bereits andere Füllstoffe und Additive enthalten können.

19. Verfahren nach mindestens einem der Ansprüche 9 bis 18,
**dadurch gekennzeichnet, dass** die Agglomeratpartikel kugelförmig sind.

20. Verfahren nach mindestens einem der Ansprüche 9 bis 19,
**dadurch gekennzeichnet, dass** die Agglomeratpartikel hohl oder kompakt sind.

21. Verfahren nach mindestens einem der Ansprüche 9 bis 20,
**dadurch gekennzeichnet, dass** die Agglomeratpartikel in einer rieselfähigen nicht staubenden Form vorliegen.

22. Verwendung der Agglomeratpartikel nach mindestens einem der Ansprüche 1 bis 8 zur Herstellung von Nanodispersionen, Nanokompositen und/oder Nanobeschichtungen.

23. Verwendung nach Anspruch 22 zur Herstellung von Lacken, Farben, Tinten, Beschichtungssystemen, Flammschutzsystemen und/oder elektrorheologischen Flüssigkeiten.

24. Verwendung nach Anspruch 22 zur Herstellung von Kompositen für dielektrische, elektrooptische, optische und elektrische Applikationen sowie medizinische Anwendungen.

25. Verwendung nach Anspruch 22 als kosmetischer, pharmazeutischer oder medizinischer Hilfsstoff.

## Claims

1. Agglomerate particles having an average particle diameter of 0.5 to 100 µm comprising agglomerated nanoscale primary particles selected form oxides of the following elements: silicon, tin, titanium, zirconium, calcium, strontium, barium, aluminium, yttrium, zinc, tantalum, cerium, gadolinium, holmium, erbium, ytterbium and also glass, glass ceramic and ceramic-forming materials and/or combinations thereof having an average particle size in the range of 2 to 500 nm.

2. Agglomerate particles according to claim 1, **characterised in that** they are spherical, preferably ball-shaped.

3. Agglomerate particles according to claim 1 or 2, **characterised in that** the nanoscale primary particles are spherical preferably ball-shed

4. Agglomerate particles according to at least one of the claims 1 to 3, **characterised in that** the nanoscale primary particles essentially have the same particle size.

5. Agglomerate particles according to at least one of the claims 1 to 4, **characterised in that** the average particle diameter of the agglomerate particles s in the range of 1 to 20 µm.

6. Agglomerate particles according to at least one of the claims 1 to 5, **characterised in that** the nanoscale primary particles are functionalised in their surface.

7. Agglomerate particles according to claim 6, **characterised in that** the surface is modified with silanes, silazanes, siloxanes, carboxylic acids, alcohols, amines, amino acids, sulphonates, (poly)phosphates, ampholytes, thermoplastic homopolymers, (block)copolymers and/or combinations thereof.

8. Agglomerate particles according to at least one of the claims 1 to 8, **characterised in that** the agglomerate particles are hollow or compact.

9. Method for incorporation of agglomerate particles according to at least one of the preceding claims in a polymer matrix, **characterised in that** agglomerate particles with a particle diameter of 0.5 to 50 µm which are formed by agglomeration of nanoscale primary particles with a particle diameter of 0.5 to 100 nm are incorporated in the polymer matrix in solid form.

10. Method according to claim 9, **characterised in that** the agglomerate particles have a diameter of 1 to 20 µm.

11. Method according to claim 9 or 10, **characterised in that** up to 40% by weight, preferably up to 25% by weight, of agglomerate particles, relative to the total mass of the polymer matrix are incorporated.

12. Method according to at least one of the claims 9 to 11, **characterised in that** the agglomerate particles or the agglomerates thereof are present in a master batch.

13. Method according to at least one of the claims 9 to 12, **characterised in that** the agglomerate particles are chosen such that they disintegrate by means of shear forces into the nanoscale primary particles during incorporation in the polymer matrix or a polymer precursor.

14. Method according to claim 13, **characterised in that** in addition an energy supply is effected, e.g. by ultrasound.

15. Method according to at least one of the claims 9 to 14, **characterised in that** the incorporation of the agglomerate particles is effected by means of mixer, kneaders, dissolvers or by means of extrusion in an extruder

16. Method according to one of the claims 13 to 15, **characterised in that** the agglomerate particles are formed by agglomeration of surface-functionalised, nanoscale primary particles.

17. Method according to at least one of the claims 9 to 16, **characterised in that** the agglomeration of the primary particles into agglomerate particles is effected by spray drying, spray freeze drying, freeze drying and/ or aerosol methods.

18. Method according to at least one of the claims 6 to 17, **characterised in that** the polymer matrix is selected from polymamides **in that** the polymer matrix is selected from polymamides, polyurethanes, polyesters (saturated, unsaturated and polycarobnates), polyacrilonitriles, butadiene- or isoprene polymers, polystyrenes, polypropylene, polyethylene, polyvinylchloride, poly(meth)acrlyates, polyethers, silicones, inorganic-organic hybrid polymers (ORMOCERs), non-shrinking or expanding polymers, such as spiroorthoesters, electrically conductive polymers, aminoplasts, melamine resins, polysulphones, polyimides, the combinations thereof to form copolymers, blends or, derived therefrom, composites and solvent or reactive monomer-containing (paint) systems which can already contain if necessary other fillers and additives.

19. Method according to at least one of the claims 9 to 18, **characterised in that** the agglomerate particles are spherical.

20. Method according to at least one of the claims 9 to 19, **characterised in that** the agglomerate particles are hollow or compact

21. Method according to at least one of the claims 9 to 20, **characterised in that** the agglomerate particles are present in a flowable, non-dusty form.

22. Use of the agglomerate particles according to at least one of the claims 1 to 8 for the production of nanodispersions, naoncomposites and/or nanocoatings.

23. Use according to claim 22 for the production of varnishes, paints, inks, coating systems, flame retardant systems and/or electrorheolgical liquids.

24. Use according to claim 22 for the production of composites for dielectric, electrooptical, optical and electrical applications and also medical applications.

25. Use according to claim 22 as a cosmetic, pharmaceutical or medical aid.

## Revendications

1. Particules agglomérées ayant un diamètre moyen de particule de 0,5 à 100 µm, constituées de particules primaires agglomérées à l'échelle nanométrique, choisies parmi les oxydes des éléments suivants : silicium, étain, titane, zirconium, calcium, strontium, baryum, aluminium, yttrium, zinc, tantale, cérium, gadolinium, holmium, erbium, ytterbium, ainsi que le verre, la vitrocéramique et les matériaux formant une céramique et/ou leurs combinaisons, ayant une granulométrie moyenne comprise dans la plage de 2 à 500 nm.

2. Particules agglomérées selon la revendication 1, **caractérisées en ce qu'**elles sont sphériques et ont de préférence la forme d'une boule.

3. Particules agglomérées selon la revendication 1 ou 2, **caractérisées en ce que** les particules primaires à l'échelle nanométrique sont sphériques, et ont de préférence la forme d'une boule.

4. Particules agglomérées selon au moins l'une des revendications 1 à 3, **caractérisées en ce que** les particules primaires à l'échelle nanométrique ont sensiblement la même granulométrie.

5. Particules agglomérées selon au moins l'une des revendications 1 à 4, **caractérisées en ce que** le diamètre moyen des particules agglomérées est compris dans la plage de 1 à 20 µm.

6. Particules agglomérées selon au moins l'une des revendications 1 à 5, **caractérisées en ce que** les particules primaires à l'échelle nanométrique sont fonctionnalisées en surface.

7. Particules agglomérées selon la revendication 6, **caractérisées en ce que** leur surface est modifiée par des silanes, des silazanes, des siloxanes, des acides carboxyliques, des alcools, des amines, des acides aminés, des sulfonates, des (poly)phosphates, des ampholytes, des homopolymères thermoplastiques, des copolymères (à blocs) et/ou leurs combinaisons.

8. Particules agglomérées selon au moins l'une des revendications 1 à 7, **caractérisées en ce que** les particules agglomérées sont creuses ou compactes.

9. Procédé pour incorporer dans une matrice polymère des particules agglomérées selon l'une des revendications précédentes, **caractérisé en ce qu'**on incorpore dans la matrice polymère des particules agglomérées, ayant un diamètre de particule de 0,5 à 50 µm, formées par agglomération de particules primaires à l'échelle nanométrique ayant un diamètre de particule de 0,5 à 100 nm.

10. Procédé selon la revendication 9, **caractérisé en ce que** les particules agglomérées ont un diamètre de 1 à 20 µm.

11. Procédé selon la revendication 9 ou 10, **caractérisé en ce qu'**on incorpore jusqu'à 40 % en poids, de préférence jusqu'à 25 % en poids de particules agglomérées, par rapport à la masse totale de la matrice polymère.

12. Procédé selon au moins l'une des revendications 9 à 11, **caractérisé en ce que** les particules agglomérées ou leurs agglomérats sont présents dans un mélange-maître.

13. Procédé selon au moins l'une des revendications 9 à 12, **caractérisé en ce que** les particules agglomérées sont choisies de façon qu'elles se décomposent sous l'effet de forces de cisaillement en les particules primaires à l'échelle nanométrique lors de leur incorporation dans la matrice polymère ou dans un précurseur de polymère.

14. Procédé selon la revendication 13, **caractérisé en ce qu'**on procède en outre à un apport d'énergie, par exemple par ultrasons.

15. Procédé selon au moins l'une des revendications 9 ou 14, **caractérisé en ce que** l'incorporation des particules agglomérées s'effectue à l'aide de mélangeurs, de malaxeurs, de dissolveurs, ou par extrusion dans une extrudeuse.

16. Procédé selon au moins l'une des revendications 13 à 15, **caractérisé en ce que** les particules agglomérées sont formées par agglomération de particules primaires à l'échelle nanométrique fonctionnalisées en surface.

17. Procédé selon au moins l'une des revendications 9 à 16, **caractérisé en ce que** l'agglomération des particules primaires en particules agglomérées a lieu par séchage par atomisation, lyophilisation par pulvérisation, lyophilisation et/ou par un procédé en aérosol.

18. Procédé selon au moins l'une des revendications 9 à 17, **caractérisé en ce que** la matrice polymère est choisie parmi les polyamides, les polyuréthannes, les polyesters (saturés, insaturés et polycarbonates), les polyacrylonitriles, les polymères du butadiène ou de l'isoprène, les polystyrènes, le polypropylène, le polyéthylène, le poly(chlorure de vinyle), les poly(méth)acrylates, les polyéthers, les silicones, les polymères hybrides inorganiques-organiques (ORMOCER), les polymères non rétractables ou expansibles, tels que les spiroorthoesters, les polymères conducteurs de l'électricité, les aminoplastes, les résines de mélamine, les polysulfones, les polyimides, leurs combinaisons en copolymères, les mélanges ou composés qui en dérivent, et les systèmes (de vernis) contenant des solvants ou des monomères réactifs, qui éventuellement peuvent déjà contenir d'autres matières de charge et additifs.

19. Procédé selon au moins l'une des revendications 9 à 18, **caractérisé en ce que** les particules agglomérées ont la forme d'une boule.

20. Procédé selon au moins l'une des revendications 9 à 19, **caractérisé en ce que** les particules agglomérées sont creuses ou compactes.

21. Procédé selon au moins l'une des revendications 9 à 20, **caractérisé en ce que** les particules agglomérées se présentent sous une forme à écoulement libre sans formation de poussières.

22. Utilisation des particules agglomérées selon au moins l'une des revendications 1 à 8 pour fabriquer des nano-dispersions, des nano-composites et/ou des nano-revêtements.

23. Utilisation selon la revendication 22, pour la fabrication de vernis, de peintures, d'encres, de systèmes de revêtement, de systèmes de calorifugeage et/ou de liquides électro-rhéologiques.

24. Utilisation selon la revendication 22 pour la fabrication de composites pour applications diélectriques, électro-optiques, optiques et électriques, ainsi que pour des applications en médecine.

25. Utilisation selon la revendication 22, en tant qu'adjuvant cosmétique, pharmaceutique ou médical.
